# EUROPÄISCHE PATENTANMELDUNG

(11) **EP 1 048 945 A2**
(43) Veröffentlichungstag der Anmeldung: **02.11.2000**
(21) Anmeldenummer: 00107759.3
(22) Anmeldetag: 11.04.2000
(51) Int. Cl.: G01N 17/00, G01N 33/32, G01N 21/71, G01N 5/04

(54) **Verfahren zur Bestimmung anorganischer Bestandteile in einer Beschichtung eines Substrates**

(30) Priorität: 29.04.1999 DE 19919499
(71) Anmelder: Thyssen Krupp AG, 40211 Düsseldorf (DE)
(72) Erfinder: Brixius, Thomas, 47509 Rheurdt (DE)
(74) Vertreter: Cohausz & Florack

(57) **Zusammenfassung**

Die Erfindung betrifft ein Verfahren zur Bestimmung anorganischer Bestandteile, insbesondere Zinkpartikel, in einer organischen Matrix einer Beschichtung, die an einer anorganischen Oberfläche eines Substrates, insbesondere einem verzinkten Feinblech, fest haftet. Durch Behandlung einer beschichteten Probe des Substrates mit ionisiertem Sauerstoff bei Unterdruck wird die feste Haftung der Beschichtung vollständig aufgehoben, ohne daß dabei das Substrat selbst in Mitleidenschaft gezogen wird. Das Beschichtungsmaterial kann dann von der Oberfläche des Substrates entfernt und der Bestimmung seiner anorganischen Bestandteile mit üblichen Analysemethoden zugeführt werden.

## Beschreibung

Die Erfindung betrifft ein Verfahren zur Bestimmung anorganischer Bestandteile in einer organischen Matrix einer Beschichtung, die an einer anorganischen Oberfläche eines Substrates fest haftet.

Flachstahlbänder werden zum Zwecke des Korrosionsschutzes unter anderen durch Verzinken oberflächenveredelt. Für bestimmte Anwendungen, zum Beispiel für den Karosseriebau von Kraftfahrzeugen, werden solche verzinkten Feinbleche zusätzlich mit einem Lack beschichtet. Solche Lacke enthalten neben organischen auch anorganische Bestandteile, wie zum Beispiel Füllstoffe oder metallische Pigmente zur Verbesserung der Schweißbarkeit. Ein solcher Lack ist im Handel zum Beispiel unter dem Handelsnamen 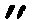 Bona-Zinc" der Firma PPG-Industries oder 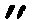 Grano-Coat" der Firma Henkel KGaA erhältlich.

In der Praxis sind beim Widerstandsschweißen von solchen lackierten verzinkten Blechen bezüglich der Schweißbarkeit wiederholt Probleme aufgetreten. Es wurde gefunden, daß diese Probleme unter anderem darauf zurückzuführen sind, daß trotz eines für die Schweißbarkeit ausreichend hohen Anteiles an Zinkpartikeln in dem Lack an der Schweißstelle die für eine gute Schweißbarkeit erforderliche Konzentration an Zinkpartikeln nicht vorhanden war. Ein solcher Fehler kann dadurch auftreten, daß der Lack vor dem Aufbringen auf das Stahlband nicht ausreichend durchmischt und damit hinsichtlich der Zinkpartikel nicht homogenisiert war. Eine gute Durchmischung des Lackes unmittelbar vor dem Auftrag ist aber erforderlich, weil der Lack mit seinen Zinkpartikeln sich unter dem Einfluß der Schwerkraft leicht entmischt.

Um im Vorfeld der Weiterverarbeitung von lackierten, verzinkten Stahlblechen durch Widerstandsschweißen festzustellen, ob ein Blech für die Verschweißbarkeit gut geeignet ist, besteht ein Bedarf für ein geeignetes Prüfverfahren.

Bekannte Verfahren für die quantitative und qualitative Bestimmung von Beschichtungen sind aus verschiedenen Gründen dafür nicht geeignet.

Bei der Untersuchung der Beschichtung mit Hilfe der Glimmlampen-Spektrometrie wird die Beschichtung atomlagenweise abgetragen. Dabei entsteht Licht mit einem charakteristischen Wellenlängenspektrum. Durch Vergleich dieses Spektrums mit entsprechenden Spektren von Referenzproben läßt sich die Konzentration der einzelnen Elemente der Beschichtung ermitteln. Dieses Verfahren setzt geeignete Referenzproben voraus, die man aber in der Regel erst selbst erstellen muß. Das bedeutet zusätzlichen Aufwand, so daß dieses Verfahren für den Einsatz in der Praxis zu umständlich ist.

Denkbar ist auch, das Beschichtungsmaterial mit organischen Lösungsmitteln oder mit Säuren abzulösen. Im Falle organischer Lösungsmittel verbleiben aber Rückstände auf dem Substrat und im Falle von Säuren, zum Beispiel konzentrierter Schwefelsäure als Lösungsmittel, wird auch das Zink der Substratoberfläche beziehungsweise die zur Verbesserung der Haftung der Beschichtung üblicherweise vorgesehene Chromat-Auflage mit angelöst. Im Ergebnis steht deshalb entweder nicht das gesamte Beschichtungsmaterial für die Analyse zur Verfügung oder das Analysenergebnis wird durch die nicht zum Beschichtungsmaterial gehörenden abgelösten Komponenten verfälscht.

Im übrigen ist es bei einem Verfahren zum Herstellen eines Flüssigkeitssensors bekannt, einen feuchtigkeitsempfindlichen Polymerfilm auf einem Substrat bereichsweise dadurch zu entfernen, daß der Polymerfilm mit einer Maske abgedeckt wird und die nicht abgedeckten Bereiche durch Behandlung mit einem Sauerstoffplasma vollständig weggeätzt werden (DE 34 16 124 A1).

Auch ist es bekannt, in einer Behandlungskammer das Verhalten eines dünnen Polymerfilms bei verschiedenen Temperaturen und Drücken unter dem Einfluß eines Oxygenplasmas zu prüfen (US 5,044,211 A).

Der Erfindung liegt die Aufgabe zugrunde, ein Verfahren zur Bestimmung anorganischer Bestandteile in einer organischen Matrix einer Beschichtung zu schaffen, das schnell und ohne großen Aufwand exakte Ergebnisse über die quantitative und qualitative Zusammensetzung der Beschichtung liefert.

Diese Aufgabe wird durch ein Verfahren der eingangs genannten Art mit folgenden Verfahrensschritten gelöst:
a) Eine Probe des Substrates wird in einer gegenüber der Umgebung abgeschlossenen Kammer bei Unterdruck mit ionisiertem Sauerstoff solange behandelt, bis die Beschichtung an dem Substrat nicht mehr fest haftet,
b) dann wird das von der Oberfläche des Substrates abgelöste Beschichtungsmaterial von dem Substrat entfernt und der Bestimmung seiner anorganischen Bestandteile zugeführt.

Durch die Behandlung der Beschichtung des Substrates mit ionisierten Sauerstoff gelingt es, die feste Haftung des Beschichtungsmateriales an der Oberfläche des Substrates vollständig aufzuheben, ohne daß von dieser Behandlung auch die Oberfläche des Substrates betroffen wird. Das lose Beschichtungsmaterial kann dann von der Oberfläche des Substrates entfernt werden, ohne daß Reste auf dem Substrat verbleiben oder von der Oberfläche des Substrates Bestandteile in das Beschichtungsmaterial gelangen. Dieser Effekt des erfindungsgemäßen Verfahrens beruht darauf, daß die anorganischen Komponenten nicht mit dem ionisierten Sauerstoff reagieren, während die organische Matrix der Beschichtung oxidiert wird. Wegen dieser Oxidation verliert die für die Haftung der Beschichtung an der anorganischen Oberfläche verantwortliche organische Matrix ihre Haftung. Damit ist sichergestellt, daß für die quantitative und/oder qualitative Bestimmung der anorganischen Bestandteile der Beschichtung das Analysenergebnis nicht verfälscht wird. Der Effekt der Erfindung, daß bei der Behandlung der Probe mit ionisiertem Sauerstoff nicht die Oberfläche des Substrates angegriffen wird, wirkt sich auch vorteilhaft bei verzinktem Stahlblech aus, das in der Regel zur Verbesserung der Haftung des Lackes mit einer Chromatschicht versehen ist.

Die Behandlung von Oberflächen eines Substrates bei Unterdruck mit ionisiertem Sauerstoff ist an sich bekannt (Zeitschrift: Stahl u. Eisen 104 (1984) Nr. 3, Seiten 141 bis 144). Bei diesem Verfahren wird eine Apparatur verwendet, die aus einem Glaszylinder besteht, in den die Stahlblechprobe in eine Probehalterung eingesetzt ist. Über eine Zuleitung wird der Kammer als Reaktions- und Trägergas Sauerstoff zugeführt. An der Kammer ist eine Vakuumpumpe angeschlossen, mit der aus der Kammer die Reaktionsgase und überschüssiger Sauerstoff abgezogen werden und der Unterdruck erzeugt wird. Die Probe ist zwischen zwei Elektroden eines Hochfrequenzgenerators angeordnet, mit dem ein Sauerstoffplasma in der Kammer erzeugt wird. Das Ziel der Behandlung einer in die Kammer eingebrachten Probe mit Sauerstoffplasma nach dem Stand der Technik ist es, die durch vorangegangene Behandlungen, wie Beizen, Walzen und Glühen, verunreinigte Oberfläche der Probe selektiv bei niedrigen Temperaturen (unter 160°C) zu reinigen.

Die Erfindung nutzt die oxidierende Wirkung des selektiv auf die Komponenten der Beschichtung wirkenden Sauerstoffplasmas aus, um die Beschichtung abzulösen, ohne daß dabei die zu bestimmenden anorganischen Bestandteile verändert werden.

Der Prozeß des Ablösens der Beschichtung von der Oberfläche des Substrates läßt sich auf einfache Art und Weise kontrollieren. Dazu ist nach einem Vorschlag der Erfindung vorgesehen, daß das Ende der für das Ablösen der Beschichtung notwendigen Behandlung mit ionisiertem Sauerstoff über den Oxidgehalt der aus der Kammer während der Behandlung abgezogenen Reaktionsgase überwacht wird.

Für die Bestimmung der anorganischen Bestandteile in dem abgelösten Beschichtungsmaterial wird vorzugsweise ein naßchemisches Analyseverfahren angewandt. Da das Beschichtungsmaterial aus säurelöslichen und säureunlöslichen Bestandteilen besteht, kann man die säureunlöslichen Bestandteile in bekannter Weise abtrennen und aufschließen, bevor sie mit Hilfe bekannter spektrometrischer Analyseverfahren (zum Beipiel optische Emissions-Spektrometrie mit induktiv gekoppeltem Plasma (ICP-OES) oder Atom Absorptions-Spektrometrie (AAS)) nach ihrem Elementgehalt bestimmt werden.

Um quantitativ den anorganischen Anteil in der Beschichtung zu bestimmen, ist nach einer weiteren Ausgestaltung der Erfindung vorgesehen, daß durch Differenzmessung des Gewichtes der Probe mit und ohne Beschichtung die Masse des Beschichtungsmateriales bestimmt wird.

Bei der Behandlung der Probe mit ionisiertem Sauerstoff ist darauf zu achten, daß ein stabiles Plasma besteht. Dies läßt sich über den Unterdruck einstellen. In Abhängigkeit unter anderem von der Größe der Kammer liegt ein für die Praxis geeigneter Unterdruck zwischen 2 und 20 hPa. Ein für die Behandlung geeigneter Temperaturbereich liegt bei ca. 50 bis 150° C.

Die nötige Behandlungszeit für das vollständige Ablösen der Beschichtung läßt sich, wie schon erwähnt, über den Oxidgehalt der aus der Kammer abgezogenen Reaktionsgase feststellen. Unabhängig davon haben aber Erfahrungen gezeigt, daß eine Behandlungszeit von 10 bis 40 Minuten ausreicht.

## Patentansprüche

1. Verfahren zur Bestimmung anorganischer Bestandteile in einer organischen Matrix einer Beschichtung, die an einer anorganischen Oberfläche eines Substrates fest haftet,
**gekennzeichnet durch** folgende Verfahrensschritte:
a) Eine Probe des Substrates wird in einer gegenüber der Umgebung abgeschlossenen Kammer bei Unterdruck mit ionisiertem Sauerstoff solange behandelt, bis die Beschichtung an dem Substrat nicht mehr fest haftet,
b) und dann wird das von der Oberfläche des Substrates abgelöste Beschichtungsmaterial von dem Substrat entfernt und der Bestimmung seiner anorganischen Bestandteile zugeführt.

2. Verfahren nach Anspruch 1,
**dadurch gekennzeichnet** , **daß** das Ende der für das Ablösen der Beschichtung notwendigen Behandlung mit ionisiertem Sauerstoff über den Oxidgehalt der aus der Kammer während der Behandlung abgezogenen Reaktionsgase überwacht wird.

3. Verfahren nach Anspruch 1 oder 2,
**dadurch gekennzeichnet** , **daß** für die Bestimmung der anorganischen Bestandteile in dem abgelösten Beschichtungsmaterial ein naßchemisches Analyseverfahren angewandt wird.

4. Verfahren nach einem der Ansprüche 1 bis 3,
**dadurch gekennzeichnet** , **daß** durch Differenzmessung des Gewichtes der Probe mit und ohne Beschichtung die Masse des Beschichtungsmateriales bestimmt wird.

5. Verfahren nach einem der Ansprüche 1 bis 4,
**dadurch gekennzeichnet** , **daß** über den Unterdruck ein stabiles Plasma des ionisierten Sauerstoffes eingestellt wird.

6. Verfahren nach Anspruch 5,
**dadurch gekennzeichnet** , **daß** der Unterdruck auf 2 bis 20 hPa eingestellt wird.

7. Verfahren nach einem der Ansprüche 1 bis 6,
**dadurch gekennzeichnet** , **daß** die Behandlungszeit der Probe 10 bis 40 Minuten beträgt.
